# EUROPEAN PATENT SPECIFICATION

(11) **EP 1 594 529 B1**
(45) Date of publication and mention of the grant of the patent: **20.01.2010**
(21) Application number: 04711811.2
(22) Date of filing: 17.02.2004
(51) Int. Cl.: A61K 38/26, C07K 14/605

(54) **ANALOGUES OF GLP-1**
GLP-1-ANALOGA
ANALOGUES DE GLP-1

(30) Priority: 19.02.2003 US 449203 P
(43) Date of publication of application: 16.11.2005
(60) Divisional application: 09156363.5
(73) Proprietor: IPSEN PHARMA, 92100 Boulogne-Billancourt (FR)
(72) Inventor: DONG, Zheng, Xin, Holliston, MA 01746 (US)
(74) Representative: Taylor, Kate Laura
(86) International application number: PCT/US2004/004421
(87) International publication number: WO 2004/074315

(56) References cited:
- EP-A- 1 227 151
- WO-A-00/34331
- WO-A-00/34332
- WO-A-01/98331
- WO-A-99/43706
- WO-A1-01/35988

## Description

### Sequence Listing

The instant application contains a "lengthy" Sequence Listing which has been submitted via four CD-R in lieu of a printed paper copy. Said CD-R, recorded on February, 10, 2004, are labeled "CRF", "Copy 1", "Copy 2" and "Copy 3" respectively, and each contains only one identical 456 Kb file (129PPCT2.APP).

### Background of the Invention

The present invention is directed to peptide analogues of glucagon-like peptide-1, the pharmaceutically-acceptable salts thereof, to methods of using such analogues to treat mammals and to pharmaceutical compositions useful therefore comprising said analogues.

Glucagon-like peptide-1 (7-36) amide (GLP-1)(SEQ ID NO: 775) is synthesized in the intestinal L-cells by tissue-specific post-translational processing of the glucagon precursor preproglucagon (Vamdell, J.M., et al., J. Histochem Cytochem, 1985:33:1080-6) and is released into the circulation in response to a meal. The plasma concentration of GLP-1 rises from a fasting level of approximately 15 pmol/L to a peak postprandial level of 40 pmol/L. It has been demonstrated that, for a given rise in plasma glucose concentration, the increase in plasma insulin is approximately threefold greater when glucose is administered orally compared with intravenously (Kreymann, B., et al., Lancet 1987:2, 1300-4). This alimentary enhancement of insulin release, known as the incretin effect, is primarily humoral and GLP-1 is now thought to be the most potent physiological incretin in humans. In addition to the insulinotropic effect, GLP-1 suppresses glucagon secretion, delays gastric emptying (Wettergren A., et al., Dig Dis Sci 1993:38:665-73) and may enhance peripheral glucose disposal (D'Alessio, D.A. et al., J. Clin Invest 1994:93:2293-6).

In 1994, the therapeutic potential of GLP-1 was suggested following the observation that a single subcutaneous (s/c) dose of GLP-1 could completely normalize postprandial glucose levels in patients with non-insulin-dependent diabetes mellitus (NIDDM) (Gutniak, M.K., et al., Diabetes Care 1994:17:1039-44). This effect was thought to be mediated both by increased insulin release and by a reduction in glucagon secretion. Furthermore, an intravenous infusion of GLP-1 has been shown to delay postprandial gastric emptying in patients with NIDDM (Williams, B., et al., J. Clin Endo Metab 1996:81:327-32). Unlike sulphonylureas, the insulinotropic action of GLP-1 is dependent on plasma glucose concentration (Holz, G.G. 4th, et al., Nature 1993:361:362-5). Thus, the loss of GLP-1-mediated insulin release at low plasma glucose concentration protects against severe hypoglycemia. This combination of actions gives GLP-1 unique potential therapeutic advantages over other agents currently used to treat NIDDM.

Numerous studies have shown that when given to healthy subjects, GLP-1 potently influences glycemic levels as well as insulin and glucagon concentrations (Orskov, C, Diabetologia 35:701-711, 1992; Holst, J.J., et al., Potential of GLP-1 in diabetes management in Glucagon III, Handbook of Experimental Pharmacology, Lefevbre PJ, Ed. Berlin, Springer Verlag, 1996, p. 311-326), effects which are glucose dependent (Kreymann, B., et al., Lancet II: 1300-1304, 1987; Weir, G.C., et al., Diabetes 38:338-342, 1989). Moreover, it Is also effective In patients with diabetes (Gutniak, M., N. Engl J Med 226:1316-1322, 1992; Nathan, D.M., et al., Diabetes Care 15:270-276, 1992), normalizing blood glucose levels in type 2 diabetic subjects (Nauck, M.A., et al., Diagbetologia 36:741-744, 1993), and improving glycemic control In type 1 patients (Creutzfeldt, W.O., et al., Diabetes Care 19:580-586, 1996), raising the possibility of its use as a therapeutic agent.

GLP-1 is, however, metabolically unstable, having a plasma half-life (t_{1/2}) of only 1-2 min *in vivo*. Exogenously administered GLP-1 is also rapidly degraded (Deacon, C.F., et al., Diabetes 44:1126-1131, 1995). This metabolic instability limits the therapeutic potential of native GLP-1, Hence, there is a need for GLP-1 analogues that are more active or are more metabolically stable than native GLP-1.
WO0198331 discloses modified glucagon-like peptide-1 (GLP-1) compounds. WO0034331 and WO0034332 disclose peptide analogues of glucagon-like peptide-1. W09943706 discloses derivatives of GLP-1 analogs having a lipophilic substituent.

### Summary of the Invention

In one aspect, the present invention is directed to a compound of formula (I),

(R²R³)-A⁷⁻A⁸-A⁹-A¹⁰-A¹¹-A¹²-A¹³-A¹⁴-A¹⁵-A¹⁶-A¹⁷⁻A¹⁸-A¹⁹-A²⁰-A²¹-A²²-A²³-A²⁴-A²⁵-A²⁶-A²⁷⁻A²⁸-A²⁹-A³⁰-A³¹-A³²-A³³-A³⁴-A³⁵-A³⁶-A³⁷⁻A³⁸-A³⁹-R¹, (I)

wherein:

A⁷ is L-His;

A⁸ is Abu, β-Ala, Ser or Val;

A⁹ is Glu;

A¹⁰ is Gly;

A¹¹ is Thr;

A¹² Is Phe;

A¹³ is Thr;

A¹⁴ is Ser;

A¹⁵ is Asp;

A¹⁶ is Val;

A¹⁷ is Ser;

A⁸ is Ser;

A¹⁹ is Tyr;

A²⁰ Is Leu;

A²¹ is Glu;

A²² is Gly;

A²³ is Gln;

A²⁴ is Ala;

A²⁵ is Ala;

A²⁸ is Lys;

A²⁷ is Glu;

A²⁸ is Phe;

A²⁹ is Ile;

A³⁰ is Ala;

A³¹ is Trp;

A³² is Leu;

A³³ is Val;

A³⁴ is Lys;

A³⁵ is β-Ala or Alb;

A³⁶ is L- or D-Arg;

A³⁷ is deleted;

A³⁸ is deleted;

A³⁹ is deleted;

R¹ is OH, NH₂, (C₁-C₃₀)alkoxy, or NH-X²-CH₂-Z⁰, wherein X² is a (C₁-C₁₂)hydrocarbon moiety, and Z⁰ is H, OH, CO₂H or CONH₂;

X³ is 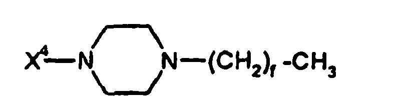 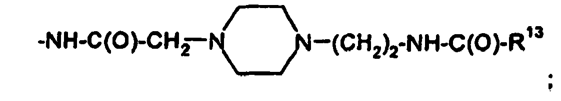 or -C(O)-NHR¹², wherein X⁴ is, independently for each occurrence, -C(O)-, -NH-C(O)- or -CH₂-, and wherein f is, independently for each occurrence, an integer from 1 to 29 inclusive;

each of R² and R³ is independently selected from the group consisting of H, (C₁-C₃₀)alkyl, (C₂-C₃₀)alkenyl, phenyl(C₁-C₃₀)alkyl, naphthyl(C₁-C₃₀)alkyl, hydroxy(C₁-C₃₀)alkyl, hydroxy(C₂-C₃₀)alkenyl, hydroxyphenyl(C₁-C₃₀)alkyl, and hydroxynaphthyl(C₁-C₃₀)alkyl; or one of R² and R³ is 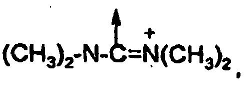 (C₁-C₃₀)acyl, (C₁-C₃₀)alkylsulfonyl, C(O)X⁵, 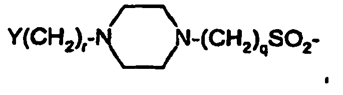 or 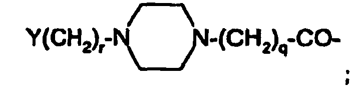 wherein Y is H, OH or NH₂; r is 0 to 4; q is 0 to 4; and X⁵ is (C₁-C₃₀)alkyl, (C₂-C₃₀)alkenyl, phenyl(C₁-C₃₀)alkyl, naphthyl(C₁-C₃₀)alkyl, hydroxy(C₁-C₃₀)alkyl, hydroxy(C₂-C₃₀)alkenyl, hydroxyphenyl(C₁-C₃₀)alkyl or hydroxynaphthyl(C₁-C₃₀)alkyl; and

R¹² and R¹³ each is, independently for each occurrence, (C₁-C₃₀)alkyl;

provided that:

(i) said compound is not

(Ser⁸, β-Ala³⁵)hGLP-1(7-36)NH₂(SEQ ID NO.779)

or a pharmaceutically acceptable salt thereof.

In a more preferred embodiment the invention features a compound according to formula (I), wherein said compound is:

(Ser⁸, Aib³⁵)hGLP-1(7-36)NH₂; (SEQ ID NO.767)

(Abu⁸, β-Ala³⁵)hGLP-1(7-36)NH₂; (SEQ ID NO.768)

(Val⁸, β-Ala³⁵)hGLP-1(7-36)NH₂; (SEQ ID NO.769)

(β-Ala^{8,35})hGLP-1(7-36)NH₂; (SEQ ID NO.770)

(Abu⁸,Alb³⁵)hGLP-1(7-36)NH₂; (SEQ ID NO.771)

(Val⁸, Alb³⁵)hGLP-1(7-36)NH₂; (SEQ ID NO.772)

or (β-Ala⁸, Alb³⁵)hGLP-1(7-36)NH₂; (SEQ ID NO.773)

or a pharmaceutically acceptable salt thereof.

In a still more preferred embodiment the invention features a compound according to formula (I), wherein said compound Is:

(Ser⁸, Aib³⁵)hGLP-1(7-36)NH₂; (SEQ ID NO.767)

(Abu⁸, β-Ala³⁵)hGLP-1(7-36)NH₂; (SEQ ID NO.768)

or (Abu⁸, Aib³⁵)hGLP-1(7-36)NH₂; (SEQ ID NO.771)

or a pharmaceutically acceptable salt thereof.

In yet a still more preferred embodiment the invention features a compound according to formula (I), wherein said compound is:

(Ser⁸, Aib³⁵)hGLP-1(7-36)NH₂; (SEQ ID NO.767)

or a pharmaceutically acceptable salt thereof.

In another aspect of the invention is featured a pharmaceutical composition comprising an effective amount of a compound according to formula (I), or a pharmaceutically acceptable salt thereof, and a pharmaceutically acceptable carrier or diluent.

In another aspect of the invention is featured use of an effective amount of a compound according to formula (I) or a pharmaceutically acceptable salt thereof in the preparation of a medicament for eliciting an agonist effect from a GLP-1 receptor in a subject in need thereof.

In another aspect of the invention is featured use of an effective amount of a compound according to formula (I) or a pharmaceutically acceptable salt thereof in the preparation of a medicament for treating a disease selected from the group consisting of Type I diabetes, Type II diabetes, obesity, glucagonomas, secretory disorders of the airway, metabolic disorder, arthritis, osteoporosis, central nervous system disease, restenosis and neurodegenerative disease, in a subject in need thereof. Preferably, said disease is Type I diabetes or Type II diabetes.

In a preferred embodiment of each of the aspects detailed in paragraphs [0953], [0955], and [0956] said compound according to formula (I) is a compound described in paragraph

through [0943], or a pharmaceutically acceptable salt thereof. Still more preferably said compound according to formula (I) is selected from among the compounds disclosed in paragraphs [0946] through [0948], or a pharmaceutically acceptable salt thereof. In a most preferred embodiment said compound according to formula (I) is (Ser⁸, Aib³⁵)hGLP-1(7-36)NH₂ (SEQ ID NO. 767), or a pharmaceutically acceptable salt thereof.

With the exception of the N-terminal amino acid, all abbreviations (e.g., Ala) of amino acids in this disclosure stand for the structure of -NH-CH(R)-CO-, wherein R is the side chain of an amino acid (e.g., CH₃ for Ala). For the N-terminal amino acid, the abbreviation stands for the structure of (R²R³)-N-CH(R)-CO-, wherein R is a side chain of an amino acid and R² and R³ are as defined above, except when A⁷ is Ura, Paa or Pta, In which case R² and R³ are not present since Ura, Paa and Pta are considered here as desamino amino acids. Certain other abbreviations for amino acids are employed herein with the following meanings:
- Abu: α-aminobutyric acid;
- Ado: 12-aminododecanoic acid;
- Aec: 4-(2-aminoethyl)-1-carboxymethyl-piperazine, represented by the structure: 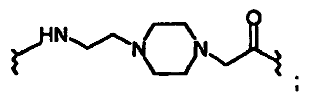
- Alb: α-aminoisobutyric acid;
- Alc: 2-aminoindane-2-carboxylic add;
- β-Ala: β-alanine;
- Amp: 4-amino-phenylalanine;
- Aun: 11-aminoundecanoic acid;
- Ava: 5-aminovaleric acid;
- Cha: cyclohexylalanine;
- Gaba: γ-aminobutyric acid;
- hArg: homoarginine;
- HEPA: 4-(2-hydroxyethyl)-1-piperazimyl acetic acid;
- HEPES: 4-(2-hydroxyethyl)-1-piperazine-ethane sulfonic acid;
- N-Me-Ala: N-methyl-alanine;
- N-Me-Asp: N-methyl-aspartic acid;
- N-Me-Glu: N-methyl-glutamic acid;
- N-Me-Gly: N-methyl-glycine;
- N^{α}-Me-His: N^{α}-methyl-histidine;
- 1-Nal: β-(1-naphthyl)alanine;
- 2-Nal: β-(2-naphthyl)alanine;
- Nle: norleucine;
- Om: ornithine;
- Paa: *trans*-3-(3-pyridyl) acrylic acid;
- 3-Pal: β-(3-pyridinyl)alanine;
- 4-Pal: β-(4-pyridinyl)alanine;
- Pta: (4-pyridylthio) acetic acid;
- Tie: *tert*-butylglycine;
- Tma-His: N,N-tetramethylamidino-histidine;
- Tba: *tert*-butylalanine; and
- Ura: urocanic acid.

What is meant by Acc is an amino acid selected from the group of 1-amino-1-cyclopropanecarboxylic acid (A3c); 1-amino-1-cyclobutanecarboxylic acid (A4c); 1-amino-1-cyclopentanecarboxylic acid (A5c); 1-amino-1-cyclohexanecarboxylic acid (A6c); 1-amino-1-cycloheptanecarboxylic acid (A7c); 1-amino-1-cyclooctanecarboxylic acid (A8c); and 1-amino-1-cyclononanecarboxylic acid (A9c).

The terms hydroxyalkyl, hydroxyphenylalkyl, and hydroxynaphthylalkyl each denotes a moiety containing 1, 2, 3, or 4 hydroxy substituents.

The term COX⁵ stands for -C=O·X⁵. Examples of -C=O·X⁵ include, but are not limited to, acetyl and phenylpropionyl.

What is meant by Lys(N^{ε}-alkanoyl) is represented by the following structure:

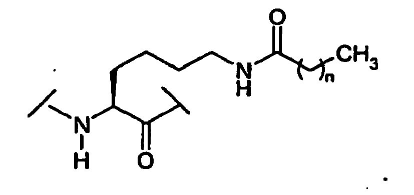

What is meant by Lys(N^{ε}-alkylsulfonyl) is represented by the following structure:

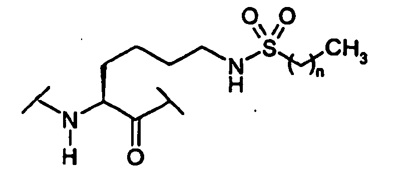

What is meant by Lys(N^{ε}-(2-(4-alkyl-1-piperazine)-acetyl)) is represented by the following structure:

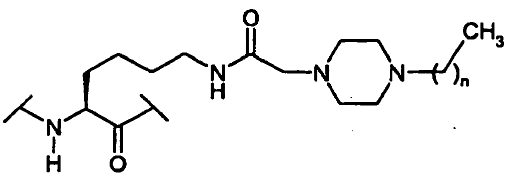

What is meant by Asp(1-(4-alkyl-piperazine)) is represented by the following structure:

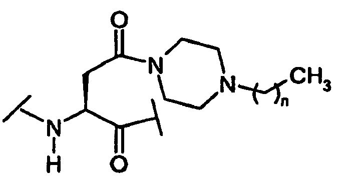

What is meant by Asp(1-alkylamino) is represented by the following structure:

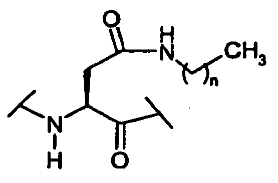

What is meant by Lys(N^{ε}-Aec-alkanoyl) is represented by the structure:

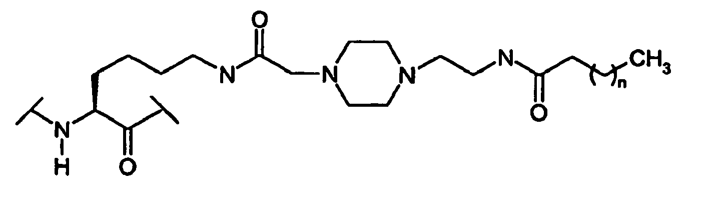

What is meant by Lys (N^{ε}-ace-alkanoyl) is represented by the structure:

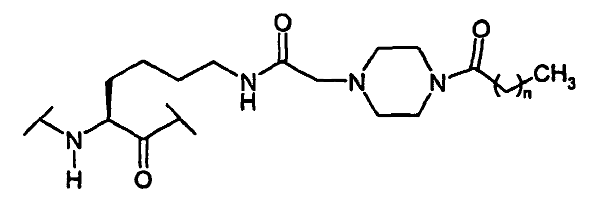

What is meant by Ser(O-alkanoyl) is represented by the structure:

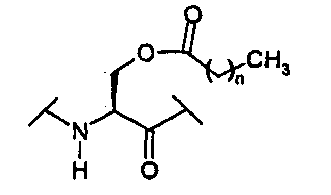

The variable n in the structure depicted for Lys(N^{ε}-alkanoyl), Lys(N^{ε}-alkylsulfonyl), Lys(N^{ε}-(2-(4-alkyl-1-piperaine)-acetyl)), Asp(1-(4-alkyl-piperazine)), Asp(1-alkylamino), Lys(N^{ε}-Aec-alkanoyl), (N^{ε}-ace-alkanoyl) and Ser(O-alkanoyl) is an integer from 1 through 30, inclusive.

The full names for other abbreviations used herein are as follows:
- Boc: t-butyloxycarbonyl;
- 2BrZ: 2-bromobenzyloxycarbonyl;
- Bzl: benzyl;
- 2CIZ: 2-chlorobenzyloxycarbonyl;
- DCM: dichloromethane;
- DIEA: diisopropylethylamine;
- DMF: dimethylformamide;
- DNP: 2,4-dinitrophenyl;
- Fm: formyl;
- Fmoc: 9-fluorenylmethoxycarbonyl;
- HBTU: 2-(1H-Benzotriazol-1-yl)-1,1,3,3-tetramethyl uronium hexafluorophosphate;
- HF: hydrogen fluoride;
- HOAc: acetic acid;
- HOBt: N-hydroxybenzotriazole;
- OcHex: O-cyclohexyl;
- PAM resin: 4-hydroxymethylphenylacetamidomethyl resin;
- TFA: trifluoroacetic acid;
- Tos: tosyl; and
- Xan: xanthyl.

The term "(C₁-C₃₀)hydrocarbon moiety" encompasses alkyl, alkenyl and alkynyl, and in the case of alkenyl and alkynyl there are C₂-C₃₀.

The term "halo" encompasses fluoro, chloro, bromo and iodo.

A peptide of this invention is also denoted herein by another format, e.g., (A5c⁸)hGLP-1(7-36)NH₂ (SEQ ID NO.774), with the substituted amino acids from the natural sequence placed between the first set of parentheses (e.g., A5c⁸ for Ala⁸ in hGLP-1). The abbreviation GLP-1 means glucagon-like peptide-1; hGLP-1 means human glucagon-like peptide-1. The numbers between the parentheses following "hGLP-1" refer to the number of amino acids present in the peptide. For example, hGLP-1(7-36) (SEQ ID NO.775) denotes amino acids 7 through 36 of the peptide sequence for human GLP-1. The sequence for hGLP-1(7-37) (SEQ ID NO.776) is listed in Mojsov, S., Int. J. Peptide Protein Res,. 40, 1992, pp. 333-342. The designation "NH₂" in hGLP-1(7-36)NH₂ indicates that the C-terminus of the peptide is amidated. hGLP-1(7-36) (SEQ ID NO.775) means that the C-terminus is the free acid. In hGLP-1(7-38) (SEQ ID NO.777), residues in positions 37 and 38 are Gly and Arg, respectively.

### Detailed Description

The peptides of this invention can be prepared by standard solid phase peptide synthesis. See, e.g., Stewart, J.M., et al., Solid Phase Synthesis (Pierce Chemical Co., 2d ed. 1984). The substituents R² and R³ of the above generic formula may be attached to the free amine of the N-terminal amino acid by standard methods known in the art. For example, alkyl groups, e.g., (C₁-C₃₀)alkyl, may be attached using reductive alkylation. Hydroxyalkyl groups, e.g., (C₁-C₃₀)hydroxyalkyl, may also be attached using reductive alkylation wherein the free hydroxy group is protected with a t-butyl ester. Acyl groups, e.g., COE¹, may be attached by coupling the free acid, e.g., E'COOH, to the free amine of the N-terminal amino acid by mixing the completed resin with 3 molar equivalents of both the free acid and diisopropylcarbodiimide in methylene chloride for one hour. If the free add contains a free hydroxy group, e.g., p-hydroxyphenylpropionic acid, then the coupling should be performed with an additional 3 molar equivalents of HOBT.

When R¹ is NH-X²-CH₂-CONH₂, (i.e., Z⁰=CONH₂), the synthesis of the peptide starts with BocHN-X²-CH₂-COOH which is coupled to the MBHA resin. If R¹ is NH-X²-CH₂-COOH, (i.e., Z⁰=COOH) the synthesis of the peptide starts with Boc-HN-X²-CH₂-COOH which is coupled to PAM resin. For this particular step, 4 molar equivalents of Boc-HN-X²-COOH, HBTU and HOBt and 10 molar equivalents of DIEA are used. The coupling time is about 8 hours.

The protected amino acid 1-(N-tert-butoxycarbonyl-amino)-l-cyclohexanecarboxylic acid (Boc-A6c-OH) was synthesized as follows. 19.1 g (0.133 mol) of 1-amino-1-cyclohexanecarboxylic acid (Acros Organics, Fisher Scientific, Pittsburgh, PA) was dissolved in 200 ml of dioxane and 100 ml of water. To this was added 67 ml of 2N NaOH and the solution was cooled in an ice-water bath. 32.0 g (0.147 mol) of di-tert-butyl-dicarbonate was added to the solution and the reaction mixture was stirred overnight at room temperature. Dioxane was then removed under reduced pressure and 200 ml of ethyl acetate was added to the remaining aqueous solution. The mixture was cooled in an ice-water bath and the pH of the aqueous layer was adjusted to about 3 by adding 4N HCl. The organic layer was separated and the aqueous layer was extracted with ethyl acetate (1 x 100 ml). The two organic layers were combined, washed with water (2 x 150 ml), dried over anhydrous MgSO₄, filtered, and concentrated to dryness under reduced pressure. The residue was recrystallized in ethyl acetate/hexanes to produce 9.2 g (0.0386 mol) of the purified product; i.e., a 29% yield from the starting material.

Boc-A5c-OH was synthesized in an analogous manner to that of Boc-A6c-OH. Other protected Acc amino acids can be prepared in an analogous manner by a person of ordinary skill in the art as enabled by the teachings herein.

In the synthesis of a GLP-1 analogue of this invention containing A5c, A6c, Aib, Boc-Lys(2ClZ)-OH and/or Boc-His(DNP)-OH, the coupling time is 2 hrs. for these residues and the residue immediately following them. Otherwise the coupling time is approximately 5 minutes. For the synthesis of (Tma-His⁷)hGLP-1(7-36)NH₂ (SEQ ID NO.778), HBTU (2 mmol) and DIEA (1.0 ml) in 4 ml DMF are used to react with the N-terminal free amine of the peptide-resin in the last coupling reaction; the coupling time is about 2 hours.

The substituents R² and R³ of the above generic formula can be attached to the free amine of the N-terminal amino acid by standard methods known in the art. For example, alkyl groups, e.g., (C₁-C₃₀)alkyl, can be attached using reductive alkylation. Hydroxyalkyl groups, e.g., (C₁-C₃₀)hydroxyalkyl, can also be attached using reductive alkylation wherein the free hydroxy group is protected with a t-butyl ester. Acyl groups, e.g., COX¹, can be attached by coupling the free acid, e.g., X¹COOH, to the free amine of the N-termlnal amino acid by mixing the completed resin with 3 molar equivalents of both the free acid and diisopropylcarbodiimide in methylene chloride for about one hour. If the free acid contains a free hydroxy group, e.g., p-hydroxyphenylpropionic acid, then the coupling should be performed with an additional 3 molar equivalents of HOBT.

A compound of the present invention can be tested for activity as a GLP-1 binding compound according to the following procedure. Cell Culture:

RIN 5F rat insulinoma cells (ATCC-# CRL-2058, American Type Culture Collection, Manassas, VA), expressing the GLP-1 receptor, were cultured in Dulbecco's modified Eagles medium (DMEM) containing 10% fetal calf serum, and maintained at about 37°C in a humidifed atmosphere of 5% CO₂/95% air. Radioligand Binding:

Membranes were prepared for radioligand binding studies by homogenization of the RIN cells in 20 ml of ice-cold 50 mM Tris-HCI with a Brinkman Polytron (Westbury, NY) (setting 6, 15 sec). The homogenates were washed twice by centrifugation (39,000 g / 10 min), and the final pellets were resuspended in 50 mM Tris-HCl, containing 2.5 mM MgCl₂, 0.1 mg/ml bacitracin (Sigma Chemical, St. Louis, MO), and 0.1% BSA. For assay, aliquots (0.4 ml) were incubated with 0.05 nM (¹²⁵I)GLP-1(7-36) (-2200 Ci/mmol, New England Nuclear, Boston, MA), with and without 0.05 ml of unlabeled competing test peptides. After a 100 min incubation (25 °C), the bound (¹²⁵I)GLP-1(7-36) (SEQ ID NO: 775) was separated from the free by rapid filtration through GF/C filters (Brandel, Gaithersburg, MD), which had been previously soaked in 0.5% polyethyleneimine. The filters were then washed three times with 5 ml aliquots of ice-cold 50 mM Tris-HCI, and the bound radioactivity trapped on the filters was counted by gamma spectrometry (Wallac LKB, Gaithersburg, MD). Specific binding was defined as the total (¹²⁵I)GLP-1(7-36) (SEQ ID NO: 775) bound minus that bound in the presence of 1000 nM GLP1(7-36) (Bachem, Torrence, CA).

The peptides of this invention can be provided in the form of pharmaceutically acceptable salts. Examples of such salts include, but are not limited to, those formed with organic acids (e.g., acetic, lactic, maleic, citric, malic, ascorbic, succinic, benzoic, methanesulfonic, toluenesulfonic, or pamoic acid), inorganic acids (e.g., hydrochloric acid, sulfuric acid, or phosphoric acid), and polymeric acids (e.g., tannic acid, carboxymethyl cellulose, polylactic, polyglycolic, or copolymers of polylactic-glycolic acids). A typical method of making a salt of a peptide of the present invention is well known in the art and can be accomplished by standard methods of salt exchange. Accordingly, the TFA salt of a peptide of the present invention (the TFA salt results from the purification of the peptide by using preparative HPLC, eluting with TFA containing buffer solutions) can be converted into another salt, such as an acetate salt by dissolving the peptide in a small amount of 0.25 N acetic acid aqueous solution. The resulting solution is applied to a semi-prep HPLC column (Zorbax, 300 SB, C-8). The column is eluted with (1) 0.1N ammonium acetate aqueous solution for 0.5 hrs., (2) 025N acetic acid aqueous solution for 0.5 hrs. and (3) a linear gradient (20% to 100% of solution B over 30 min.) at a flow rate of 4 ml/min (solution A is 0.25N acetic acid aqueous solution; solution B is 0.25N acetic acid in acetonitrile/water, 80:20). The fractions containing the peptide are collected and lyophilized to dryness.

As is well known to those skilled in the art, the known and potential uses of GLP-1 is varied and multitudinous (See, Todd, J.F., et al., Clinical Science, 1998, 95, pp. 325-329; and Todd, J.F. et al., European Journal of Clinical Investigation, 1997, 27, pp.533-536). Thus, the administration of the compounds of this invention for purposes of eliciting an agonist effect can have the same effects and uses as GLP-1 itself. These varied uses of GLP-1 may be summarized as follows, treatment of: Type I diabetes, Type II diabetes, obesity, glucagonomas, secretory disorders of the airway, metabolic disorder, arthritis, osteoporosis, central nervous system diseases, restenosis, neurodegenerative disease, renal failure, congestive heart failure, nephrotic syndrome, cirrhosis, pulmonary edema, hypertension, and disorders wherein the reduction of food intake is desired. GLP-1 analogues of the present invention that elicit an antagonist effect from a subject can be used for treating the following: hypoglycemia and malabsorption syndrome associated with gastroectomy or small bowel resection.

Accordingly, the present Invention includes within its scope pharmaceutical compositions comprising, as an active ingredient, at least one of the compounds of formula (I) in association with a pharmaceutically acceptable carrier.

The dosage of active ingredient in the compositions of this invention may be varied; however, it is necessary that the amount of the active ingredient be such that a suitable dosage form is obtained. The selected dosage depends upon the desired therapeutic effect, on the route of administration, and on the duration of the treatment. In general, an effective dosage for the activities of this invention is In the range of 1x10⁻⁷ to 200 mg/kg/day, preferably 1x10⁻⁴ to 100 mg/kg/day, which can be administered as a single dose or divided into multiple doses.

The compounds of this invention can be administered by oral, parenteral (e.g., intramuscular, intraperitoneal, intravenous or subcutaneous injection, or implant), nasal, vaginal, rectal, sublingual or topical routes of administration and can be formulated with pharmaceutically acceptable carriers to provide dosage forms appropriate for each route of administration.

Solid dosage forms for oral administration include capsules, tablets, pills, powders and granules. In such solid dosage forms, the active compound is admixed with at least one inert pharmaceutically acceptable carrier such as sucrose, lactose, or starch. Such dosage forms can also comprise, as is normal practice, additional substances other than such inert diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration include pharmaceutically acceptable emulsions, solutions, suspensions, syrups, the elixirs containing inert diluents commonly used in the art, such as water. Besides such inert diluents, compositions can also include adjuvants, such as wetting agents, emulsifying and suspending agents, and sweetening, flavoring and perfuming agents.

Preparations according to this invention for parenteral administration include sterile aqueous or non-aqueous solutions, suspensions, or emulsions. Examples of non-aqueous solvents or vehicles are propylene glycol, polyethylene glycol, vegetable oils, such as olive oil and com oil, gelatin, and Injectable organic esters such as ethyl oleate. Such dosage forms may also contain adjuvants such as preserving, wetting, emulsifying, and dispersing agents. They may be sterilized by, for example, filtration through a bacteria-retaining filter, by Incorporating sterilizing agents into the compositions, by irradiating the compositions, or by heating the compositions. They can also be manufactured in the form of sterile solid compositions which can be dissolved in sterile water, or some other sterile injectable medium immediately before use.

Compositions for rectal or vaginal administration are preferably suppositories which may contain, in addition to the active substance, excipients such as coca butter or a suppository wax.

Compositions for nasal or sublingual administration are also prepared with standard excipients well known in the art.

Further, a compound of this invention can be administered in a sustained release composition such as those described in the following patents and patent applications. U.S. Patent No. 5,672,659 teaches sustained release compositions comprising a bioactive agent and a polyester. U.S. Patent No. 5,595,760 teaches sustained release compositions comprising a bioactive agent in a gelable form. U.S. Application No. 08/929,363/(US-A-5821221) filed September 9, 1997, teaches polymeric sustained release compositions comprising a bioactive agent and chitosan. U.S. Application No. 08/740,778 (US-A-5916883) filed November 1, 1996, teaches sustained release compositions comprising a bioactive agent and cyclodextrin. U.S. Application No. 09/015.394 (WO-A-9938536) filed January 29, 1998, teaches absorbable sustained release compositions of a bioactive agent. U.S. Application No. 09/121,653 filed July 23, 1998, teaches a process for making microparticles comprising a therapeutic agent such as a peptide in an oil-in-water process. U.S. Application No. 09/131,472 filed August 10, 1998, teaches complexes comprising a therapeutic agent such as a peptide and a phosphorylated polymer. U.S. Application No. 09/184,413 filed November 2, 1998, teaches complexes comprising a therapeutic agent such as a peptide and a polymer bearing a non-polymerizable lactone. The teachings of the foregoing patents and applications are incorporated herein by reference.

Unless defined otherwise, all technical and scientific terms used herein have the same meaning as commonly understood by one of ordinary skill in the art to which this invention belongs. Also, all publications, patent applications, patents and other references mentioned herein are incorporated by reference. Synthesis:

The following example describes a synthetic method for making a peptide of the invention, which method is well-known to those skilled in the art of peptide synthesis. Where provided, the amounts of reagents are approximately those employed to carry out the synthesis on a 0.20 mmol scale. Other methods are also well known to those skilled in the art, thus the example is provided for the purpose of illustration only and is not meant to limit the scope of the present invention in any manner.

A great variety of amino acids, with and without side-chain protection, are available from a number of commercial sources. For example, the following amino acids, wherein parentheses designate the side-chain protecting group, if present, are variously available from Bachem, Torrance, CA; Nova Blochem., LaJolla, CA; Chem-Impex International; Wood Dale, IL; and Synthetech, Inc. Albany, OR: Boc-Ado-OH, Boc-Aib-OH, Boc-Ala-OH, Boc-βAla-OH, Boo-Arg(Tos)-OH, Boc-D-Arg(Tos)-OH, Boc-Asp(OcHex)-OH, Boc-D-Asp(OcHex)-OH, Boc-Aun-OH, Boc-Ava-OH, Boc-Gin-OH, Boc-Glu(OcHex)-OH, Boc-Gly-OH, Boc-His(DNP)-OH, Boc-Ile-OH, Boc-Leu-OH, Boc-Lys(2ClZ)-OH. Boc-Nal-OH, Boc-Phe-OH, Boc-Ser(Bzl)-OH, Boc-Thr(Bzl)-OH, Boc-Trp(Fm)-OH, Boc-Tyr(2BrZ)-OH, and Boc-Val-OH.

### Example 1: Synthesis of (Ser⁸, Aib³⁵)hGLP-1(7-36)NH₂

The title peptide may be synthesized on an Applied Biosystems (Foster City, CA) model 430A peptide synthesizer modified to do accelerated Boc-chemistry solid phase peptide synthesis. (See Schnolzer, et al., Int. J. Peptide Protein Res., 90:180 (1992).) 4-Methylbenzhydrylamine (MBHA) resin (Peninsula Laboratories, Belmont, CA) with the substitution of 0.91 mmol/g may be used. Boc amino acids (Bachem, CA, Torrance, CA; Nova Biochem., LaJolla, CA) may be used, e.g., with the following side chain protection: Boc-Ala-OH, Boc-Arg(Tos)-OH, Boc-Asp(OcHex)-OH, Boc-Tyr(2BrZ)-OH, Boc-His(DNP)-OH, Boc-Val-OH, Boc-Leu-OH, Boc-Gly-OH, Boc-Gln-OH, Boc-Ile-OH, Boo-Lys(2ClZ)-OH, Boc-Thr(Bzl)-OH, Boc-Ser(Bzl)-OH, Boc-Phe-OH, Boc-Glu(OcHex)-OH and Boc-Trp(Fm)-OH. The Boc groups are removed by treatment with 100% TFA for 2 x 1 min. Boc amino acids (2.5 mmol) are pre-activated with HBTU (2.0 mmol) and DIEA (1.0 mL) in 4 mL of DMF and are coupled without prior neutralization of the peptide-resin TFA salt.

At the end of the assembly of the peptide chain, the resin is treated with a solution of 20% mercaptoethanol/10% DIEA in DMF for 2 x 30 min. to remove the DNP group on the His side chain. The N-terminal Boc group is then removed by treatment with 100% TFA for 2 x 2 min. After neutralization of the peptide-resin with 10% DIEA in DMF (1 x 1 min), the formyl group on the side chain of Trp is removed by treatment with a solution of 15% ethanolamine/ 15% water/ 70% DMF for 2 x 30 min. The peptide-resin is then washed with DMF and DCM and dried under reduced pressure. The final cleavage is performed by stirring the peptide-resin in 10 mL of HF containing 1 mL of anisole and dithiothreitol (24 mg) at 0°C for approximately 75 min. HF is removed by a flow of nitrogen and the residue is then washed with ether (6 x 10 mL) and extracted with 4N HOAc (6 x 10 mL).

The peptide mixture in the aqueous extract is purified on reverse-phase preparative high pressure liquid chromatography (HPLC) using a reverse phase VYDAC® C₁₈ column (Nest Group, Southborough, MA). The column is eluted with a linear gradient (20% to 50% of solution B over 105 min.) at a flow rate of 10 mL/min (Solution A = water containing 0.1% TFA; Solution B = acetonitrile containing 0.1% of TFA). Fractions are collected and checked on analytical HPLC. Those fractions containing purified product are then combined and lyophilized to dryness. Electro-spray mass spectrometer (MS(ES)) analysis may be used to check the molecular weight of the final product.

### Examples 2 - 7

The following peptides may also be synthesized according to the procedure detailed in
Example 1, substituting appropriate amino acids during peptide assembly.
Example 2: (Abu⁸, β-Ala³⁵)hGLP-1(7-36)NH₂; (SEQ ID NO.768)
Example 3: (Val⁸, β-Ala³⁵)hGLP-1(7-36)NH₂; (SEQ ID NO.769)
Example 4: (β-Ala^{8.35})hGLP-1(7-36)NH₂; (SEQ ID NO.770)
Example 5: (Abu⁸, Aib³⁵)hGLP-1(7-36)NH₂; (SEQ ID NO.771)
Example 6: (Val⁸, Aib³⁵)hGLP-1(7-36)NH₂; and (SEQ ID NO.772)
Example 7: (β -Ala⁸, Aib³⁵)hGLP-1(7-36)NH₂; (SEQ ID NO.773)

Each of the foregoing examples can be made according to the procedures described hereinabove, with appropriate substitutions in starting materials during peptide assembly.

## Claims

1. A compound of formula (I),
(R²R³)-A⁷-A⁸-A⁹-A¹⁰-A¹¹A¹²-A¹³-A¹⁴-A¹⁵-A¹⁶-A¹⁷-A¹⁸-A¹⁹-A²⁰-A²¹-A²²A²³-A²⁴-A²⁵-A²⁵-A²⁷-A²⁸⁻A²⁹-A³⁰-A³¹-A³²-A³³-A³⁴-A³⁵-A³⁶-A³⁷-A³⁸-A³⁹-R¹, (I)
wherein:
A⁷ is L-His;
A⁸ is Abu, β-Ala, Ser or Val;
A⁹ is Glu;
A¹⁰ is Gly;
A¹¹ is Thr ;
A¹² is Phe;
A¹³ is Thr;
A¹⁴ is Ser;
A¹⁵ is Asp;
A¹⁶ is Val;
A¹⁷ is Ser;
A¹⁸ is Ser;
A¹⁹ is Tyr;
A²⁰ is Leu;
A²¹ is Glu;
A²² is Gly;
A²³ is Gln;
A²⁴ is Ala;
A²⁵ is Ala;
A²⁸ is Lys;
A²⁷ is Glu;
A²⁸ is Phe;
A²⁹ is lie;
A³⁰ is Ala;
A³¹ is Trp;
A³² is Leu;
A³³ is Val;
A³⁴ is Lys;
A³⁵ is β-Ala or Aib;
A³⁸ is L- or D-Arg;
A³⁷ is deleted;
A³⁸ is deleted;
A³⁹ is deleted;
R¹ is OH, NH₂, (C₁-C₃₀)alkoxy, or NH-X²-CH₂-Z⁰, wherein X² is a (C₁-C₁₂)hydrocarbon moiety, and Z⁰ is H, OH, CO₂H or CONH₂;
X³ is 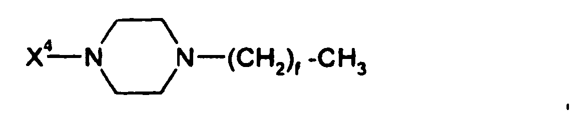 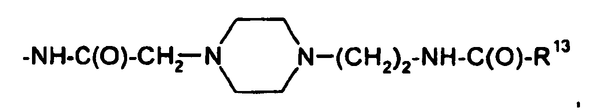 or -C(O)-NHR¹², wherein X⁴ is, independently for each occurrence, -C(O)-, -NH-C(O)- or -CH₂-, and wherein f is, independently for each occurrence, an integer from 1 to 29 inclusive; each of R² and R³ is independently selected from the group consisting of H, (C₁-C₃₀)alkyl, -(C₂-C₃₀)alkenyl, phenyl(C₁-C₃₀)alkyl, naphthyl(C₁-C₃₀)alkyl, hydroxy(C₁-C₃₀)alkyl, hydroxy(C₂-C₃₀)alkenyl, hydroxyphonyl(C₁-C₃₀)alkyl, and hydroxynaphthyl(C₁-C₃₀)alkyl; or one of R² and
R³ is 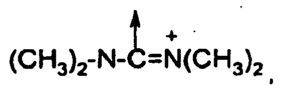 (C₁-C₃₀)acyl, (C₁-C₃₀)alkylsulfonyl, C(O)X⁵, 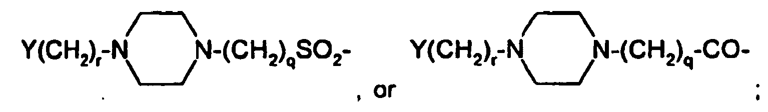 wherein Y is H, OH or NH₂; r is 0 to 4; q is 0 to 4; and X⁵ is (C₁-C₃₀)alkyl, (C₂-C₃₀)alkenyl, phenyl(C₁-C₃₀)alkyl, naphthyl(C₁-C₃₀)alkyl, hydroxy(C₁-C₃₀)alkyl, hydroxy(C₂-C₃₀)alkenyl, hydroxyphenyl(C₁-C₃₀)alkyl or hydroxynaphthyl(C₁-C₃₀)alkyl;
and
R¹² and R¹³ each is, independently for each occurrence, (C₁-C₃₀)alkyl; provided that:
(i) said compound is not:
(Ser⁸, β-Ala³⁵)hGLP-1(7-36)NH₂(SEQ ID NO: 779);
or a pharmaceutically acceptable salt thereof.

2. A compound according to claim 1, wherein said compound is:
(Ser⁸, Aib³⁵)hGLP-1(7-36)NH₂; (SEQ ID NO.767)
(Abu⁸, β-Ala³⁵)hGLP-1(7-36)NH₂; (SEQ ID NO.768)
(Val⁸, β-Ala³⁵)hGLP-1(7-36)NH₂; (SEQ ID NO.769)
(β-Ala^{8,35})hGLP-1(7-36)NH₂; (SEQ ID NO.770)
(Abu⁸, Aib³⁵)hGLP-1(7-36)NH₂: (SEQ ID NO.771)
(Val⁸, Aib³⁵)hGLP-1(7-36)NH₂; (SEQ ID NO.772)
or (β-Ala⁸, Aib³⁵)hGLP-1(7-36)NH₂; (SEQ ID NO.773)
or a pharmaceutically acceptable salt thereof.

3. A compound according to claim 2, wherein said compound is:
(Ser⁸, Aib³⁵)hGLP-1(7-36)NH₂; (SEQ ID NO.767)
(Abu⁸, β-Ala³⁵)hGLP-1(7-36)NH₂; (SEQ ID NO.768)
or (Abu⁸, Aib³⁵)hGLP-1(7-36)NH2; (SEQ ID NO.771) or a pharmaceutically acceptable salt thereof.

4. A compound according to claim 3, wherein said compound is:
(Ser⁸, Aib³⁵)hGLP-1(7-36)NH₂; (SEQ ID NO.767) or a pharmaceutically acceptable salt thereof.

5. A pharmaceutical composition comprising an effective amount of a compound according to claim 1 or a pharmaceutically acceptable salt thereof and a pharmaceutically acceptable carrier or diluent.

6. A pharmaceutical composition according to claim 5, wherein said compound is:
(Ser⁸, Aib³⁵)hGLP-1 (7-36)NH₂; (SEQ ID NO.767)
(Abu⁸, β-Ala³⁵)hGLP-1(7-36)NH₂; (SEQ ID NO.768)
(Val⁸, β-Ala³⁵)hGLP-1(7-36)NH₂; (SEQ ID NO.769)
(β-Ala^{8,35})hGLP-1(7-36)NH₂; (SEQ ID NO.770)
(Abu⁸, Aib³⁵)hGLP-1(7-36)NH₂; (SEQ ID NO.771)
(Val⁸, Aib³⁵)hGLP-1(7-36)NH₂; (SEQ ID NO.772)
or (β-Ala⁸, Aib³⁵)hGLP-1(7-36)NH₂; (SEQ ID NO.773)
or a pharmaceutically acceptable salt thereof.

7. A pharmaceutical composition according to claim 6, wherein said compound is:
(Ser⁸, Aib³⁵)hGLP-1(7-36)NH₂; (SEQ ID NO.767)
(Abu⁸, β-Ala³⁵)hGLP-1(7-36)NH₂; (SEQ ID NO.768)
or(Abu⁸, Aib³⁵)hGLP-1(7-36)NH₂; (SEQ ID NO.771)
or a pharmaceutically acceptable salt thereof.

8. A pharmaceutical composition according to claim 7, wherein said compound is:
(Ser⁸, Aib³⁵)hGLP-1(7-36)NH₂; (SEQ ID NO.767)
or a pharmaceutically acceptable salt thereof.

9. Use of an effective amount of a compound according to claim 1 or a pharmaceutically acceptable salt thereof in the preparation of a medicament for eliciting an agonist effect from a GLP-1 receptor in a subject in need thereof.

10. Use of an effective amount of a compound according to claim 1 or a pharmaceutically acceptable salt thereof in the preparation of a medicament for treating a disease selected from the group consisting of Type I I diabetes, Type II diabetes, obesity, glucagonomas, secretory disorders of the airway, metabolic disorder, arthritis, osteoporosis, central nervous system disease, restenosis and neurodegenerative disease, in a subject in need thereof..

11. Use according to claim 10 wherein said disease is Type I diabetes or Type II diabetes.

12. Use according to any one of claims 9 to 11, wherein said compound is:
(Ser⁸, Aib³⁵)hGLP-1(7-36)NH₂; (SEQ ID NO.767)
(Abu⁸, β-Ala³⁵)hGLP-1(7-36)NH₂; (SEQ ID NO.768)
(Val⁸, β-Ala³⁵)hGLP-1(7-36)NH₂; (SEQ ID NO.769)
(β-Alae^{8,35})hGLP-1(7-36)NH₂; (SEQ ID NO.770)
(Abu⁸, Aib³⁵)hGLP-1(7-36)NH₂; (SEQ ID NO.771)
(Vai⁸, Aib³⁵)hGLP-1(7-36)NH₂; (SEQ ID NO.772)
or (β-Ala⁸, Aib³⁵)hGLP-1(7-36)NH₂; (SEQ ID NO.773) or a pharmaceutically acceptable salt thereof.

13. Use according to claim 12, wherein said compound is:
Ser⁸, Aib³⁵)hGLP-1(7-36)NH₂; (SEQ ID NO.767)
(Abu⁸, β-Ala³⁵)hGlP-1(7-36)NH₂: (SEQ ID NO.768)
or (Abu⁸, Aib³⁵)hGLP-1(7-36)NH₂; (SEQ ID NO.771) or a pharmaceutically acceptable salt thereof.

14. Use according to claim 13, wherein said compound is:
(Ser⁸, Aib³⁵)hGLP-1(7-36)NH₂; (SEQ ID NO.767)
or a pharmaceutically acceptable salt thereof.

## Patentansprüche

1. Verbindung der Formel (I),
(R²R³) -A⁷⁻A⁸-A⁹-A¹⁰-A¹¹-A¹²-A¹³-A¹⁴-A¹⁵-A¹⁶-A¹⁷-A¹⁸-A¹⁹-A²⁰-A²¹-A²²-A²³-A²⁴-A²⁵-A²⁶-A²⁷-A²⁸-A²⁹-A³⁰-A³¹-A³²-A³³-A³⁴-A³⁵-A³⁶-A³⁷-A³⁸-A³⁹-R¹, (I)
in der:
A⁷ L-His ist;
A⁸ Abu, β-Ala, Ser oder Val ist;
A⁹ Glu ist;
A¹⁰ Gly ist;
A¹¹ Thr ist;
A¹² Phe ist;
A¹³ Thr ist;
A¹⁴ Ser ist;
A¹⁵ Asp ist;
A¹⁶ Val ist;
A¹⁷ Ser ist;
A¹⁸ Ser ist;
A¹⁹ Tyr ist;
A²⁰ Leu ist;
A²¹ Glu ist;
A²² Gly ist;
A²³ Gln ist
A²⁴ Ala ist;
A²⁵ Ala ist;
A²⁶ Lys ist;
A²⁷ Glu ist;
A²⁸ Phe ist;
A²⁹ Ile ist;
A³⁰ Ala ist;
A³¹ Trp ist;
A³² Leu ist;
A³³ Val ist;
A³⁴ Lys ist;
A³⁵ β-Ala oder Aib ist;
A³⁶ L- oder D-Arg ist;
A³⁷ gestrichen ist;
A³⁸ gestrichen ist;
A³⁹ gestrichen ist;
R¹ OH, NH₂, (C₁-C₃₀)-Alkoxy oder NH-X²-CH₂-Z⁰ ist, wobei X² ein (C₁-C₁₂)-Kohlenwasserstoffanteil ist und Z⁰ H, OH, CO₂H oder CONH₂ ist;
X³ 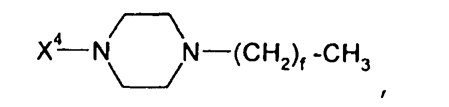 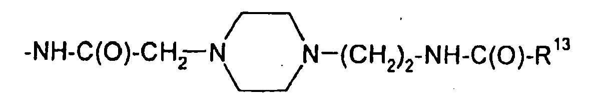 oder -C(O)-NHR¹² ist, wobei X⁴ unabhängig für jedes Auftreten -C(O)-, -NH-C(O)- oder -CH₂- ist und wobei f unabhängig für jedes Auftreten eine Zahl von 1 bis einschließlich 29 ist;
jedes von R² und R³ unabhängig voneinander aus der Gruppe bestehend aus H, (C₁-C₃₀) -Alkyl, (C₂-C₃₀) -Alkenyl, Phenyl-(C₁-C₃₀)-alkyl, Naphthyl- (C₁-C₃₀) -alkyl, Hydroxy- (C₁-C₃₀)-alkyl, Hydroxy(C₂-C₃₀)-alkenyl, Hydroxyphenyl-(C₁-C₃₀)-alkyl und Hydroxynaphthyl-(C₁-C₃₀)-alkyl ausgewählt ist; oder eines von R² und R³ 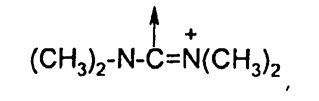 (C₁-C₃₀)-Acyl, (C₁-C₃₀)-Alkylsulfonyl, C(O)X⁵, 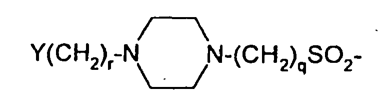 oder 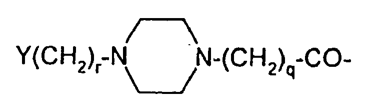 ist,
wobei Y H, OH oder NH₂ ist, r 0 bis 4 ist, q 0 bis 4 ist und X⁵ (C₁-C₃₀)-Alkyl, (C₂-C₃₀)-Alkenyl, Phenyl-(C₁-C₃₀)-alkyl, Naphthyl-(C₁-C₃₀)-alkyl, Hydroxy-(C₁-C₃₀)-alkyl, Hydroxy- (C₂-C₃₀) -alkenyl, Hydroxyphenyl- (C₁-C₃₀) -alkyl oder Hydroxynaphthyl-(C₁-C₃₀)-alkyl ist;
und
jedes von R¹² und R¹³ unabhängig für jedes Auftreten (C₁-C₃₀)-Alkyl ist;
mit der Maßgabe, dass die Verbindung nicht (Ser⁸, β-Ala)hGLP-1(7-36)NH₂ (SEQ ID NO: 779) ist; oder ein pharmazeutisch akzeptables Salz davon ist.

2. Verbindung nach Anspruch 1, wobei die Verbindung
(Ser⁸, Aib³⁵)hGLP-1(7-36)NH₂, (SEQ ID NO: 767)
(Abu⁸, β-Ala³⁵)hGLP-1(7-36)NH₂, (SEQ ID NO: 768)
(Val⁸, β-Ala³⁵)hGLP-1(7-36)NH₂, (SEQ ID NO: 769)
(β-Ala^{8,35}) hGLP-1(7-36)NH₂, (SEQ ID NO: 770)
(Abu⁸, Aib³⁵)hGLP-1(7-36)NH₂, (SEQ ID NO: 771)
(Val⁸, Aib³⁵)hGLP-1(7-36)NH₂ (SEQ ID NO: 772)
oder (β-Ala⁸, Aib³⁵)hGLP-1(7-36)NH₂ ist (SEQ ID NO: 773)
oder ein pharmazeutisch akzeptables Salz davon ist.

3. Verbindung nach Anspruch 2, wobei die Verbindung
(Ser⁸, Aib³⁵)hGLP-1(7-36)NH₂, (SEQ ID NO: 767)
(Abu⁸, β-Ala³⁵)hGLP-1(7-36)NH₂ (SEQ ID NO: 768)
oder (Abu⁸, Aib³⁵)hGLP-1(7-36)NH₂ ist (SEQ ID NO: 771)
oder ein pharmazeutisch akzeptables Salz davon ist.

4. Verbindung nach Anspruch 3, wobei die Verbindung
(Ser⁸, Aib³⁵)hGLP-1(7-36)NH₂ ist (SEQ ID NO: 767)
oder ein pharmazeutisch akzeptables Salz davon ist.

5. Pharmazeutische Zusammensetzung, die eine wirksame Menge einer Verbindung gemäß Anspruch 1 oder eines pharmazeutisch akzeptablen Salzes davon und einen pharmazeutisch akzeptablen Träger oder ein pharmazeutisch akzeptables Verdünnungsmittel enthält.

6. Pharmazeutische Zusammensetzung nach Anspruch 5, wobei die Verbindung
(Ser⁸, Aib³⁵)hGLP-1(7-36)NH_{2,} (SEQ ID NO: 767)
(Abu⁸, β-Ala³⁵)hGLP-1(7-36)NH₂, (SEQ ID NO: 768)
(Val⁸, β-Ala³⁵)hGLP-1(7-36)NH₂, (SEQ ID NO: 769)
(β-Ala^{8,35})hGLP-1(7-36)NH₂, (SEQ ID NO: 770)
(Abu⁸, Aib³⁵)hGLP-1(7-36)NH₂, (SEQ ID NO: 771)
(Val⁸, Aib³⁵)hGLP-1(7-36)NH₂ (SEQ ID NO: 772)
oder (β-Ala⁸, Aib³⁵)hGLP-1(7-36)NH₂ ist (SEQ ID NO: 773)
oder ein pharmazeutisch akzeptables Salz davon ist.

7. Pharmazeutische Zusammensetzung nach Anspruch 6, wobei die Verbindung
(Ser⁸, Aib³⁵)hGLP-1(7-36)NH₂, (SEQ ID NO: 767)
(Abu⁸, β-Ala³⁵)hGLP-1(7-36)NH₂ (SEQ ID NO: 768)
oder (Abu⁸, Aib³⁵)hGLP-1(7-36)NH₂ ist (SEQ ID NO: 771)
oder ein pharmazeutisch akzeptables Salz davon ist.

8. Pharmazeutische Zusammensetzung nach Anspruch 7, bei der die Verbindung
(Ser⁸, Aib³⁵)hGLP-1(7-36)NH₂ ist (SEQ ID NO: 767)
oder ein pharmazeutisch akzeptables Salz davon ist.

9. Verwendung einer wirksamen Menge einer Verbindung gemäß Anspruch 1 oder eines pharmazeutisch akzeptablen Salzes davon bei der Herstellung eines Medikaments zur Auslösung eines Agonisteneffekts bei einem GLP-1-Rezeptor eines bedürftigen Subjekts.

10. Verwendung einer wirksamen Menge einer Verbindung gemäß Anspruch 1 oder eines pharmazeutisch akzeptablen Salzes davon bei der Herstellung eines Medikaments zur Behandlung einer aus der Gruppe bestehend aus Diabetes Typ I, Diabetes Typ 2, Fettsucht, einem Glukagonom, Parasekretionen der Atemwege, Stoffwechselstörung, Arthrose, Osteoporose, Erkrankung des zentralen Nervensystems, Restenose und neurodegenerativer Erkrankung ausgewählten Erkrankung eines bedürftigen Subjekts.

11. Verwendung nach Anspruch 10, bei der die Erkrankung Diabetes Typ I oder Diabetes Typ 2 ist.

12. Verwendung nach einem der Ansprüche 9 bis 11, bei der die Verbindung
(Ser⁸, Aib³⁵)hGLP-1(7-36)NH₂, (SEQ ID NO: 767) (Abu⁸, β-Ala³⁵)hGLP-1(7-36)NH₂, (SEQ ID NO: 768) (Val⁸, β-Ala³⁵)hGLP-1(7-36)NH₂, (SEQ ID NO: 769) (β-Alae^{8,35})hGLP-1(7-36)NH₂, (SEQ ID NO: 770) (Abu⁸, Aib³⁵)hGLP-1(7-36)NH₂, (SEQ ID NO: 771) (Val⁸, Aib³⁵)hGLP-1(7-36)NH₂ (SEQ ID NO: 772) oder (β-Ala⁸, Aib³⁵) hGLP-1 (7-36) NH₂ ist (SEQ ID NO: 773) oder ein pharmazeutisch akzeptables Salz davon ist.

13. Verwendung nach Anspruch 12, bei der die Verbindung
(Ser⁸, Aib³⁵)hGLP-1(7-36)NH₂, (SEQ ID NO: 767) (Abu⁸, β-Ala³⁵)hGLP-1(7-36)NH₂ (SEQ ID NO: 768) oder (Abu⁸, Aib³⁵)hGLP-1(7-36)NH₂ ist (SEQ ID NO: 771) oder ein pharmazeutisch akzeptables Salz davon ist.

14. Verwendung nach Anspruch 13, bei der die Verbindung (Ser⁸, Aib³⁵)hGLP-1(7-36)NH₂ ist (SEQ ID NO: 767) oder ein pharmazeutisch akzeptables Salz davon ist.

## Revendications

1. Composé représenté par la formule (I),
(R²R³)-A⁷-A⁸-A⁹-A¹⁰-A¹¹-A¹²-A¹³-A¹⁴-A¹⁵-A¹⁶-A¹⁷-A¹⁸-A¹⁹-A²⁰-A²¹-A²²-A²³-A²⁴-A²⁵-A²⁶-A²⁷-A²⁸-A²⁹-A³⁰-A³¹-A³²-A³³-A³⁴-A³⁵-A³⁶-A³⁷-A³⁸-A³⁹-R¹ (I)
dans laquelle :
A⁷ est L-His ;
A⁸ est Abu, β-Ala, Ser ou Val ;
A⁹ est Glu ;
A¹⁰ est Gly ;
A¹¹ est Thr ;
A¹² est Phe ;
A¹³ est Thr ;
A¹⁴ est Ser ;
A¹⁵ est Asp ;
A¹⁶ est Val ;
A¹⁷ est Ser ;
A¹⁸ est Ser ;
A¹⁹ est Tyr ;
A²⁰ est Leu ;
A²¹ est Glu ;
A²² est Gly ;
A²³ est Gln;
A²⁴ est Ala ;
A²⁵ est Ala ;
A²⁶ est Lys ;
A²⁷ est Glu ;
A²⁸ est Phe ;
A²⁹ est Ile ;
A³⁰ est Ala ;
A³¹ est Trp ;
A³² est Leu ;
A³³ est Val ;
A³⁴ est Lys ;
A³⁵ est β-Ala ou Aib ;
A³⁶ est L- ou D-Arg ;
A³⁷ est délété ;
A³⁸ est délété ;
A³⁹ est délété ;
R¹ est OH, NH₂, alcoxy en C₁-C₃₀, ou NH-X₂-CH₂-Z⁰, où X² est une fraction hydrocarbonée en C₁-C₁₂ et Z⁰ est H, OH, CO₂H
ou CONH₂ ;
X³ est 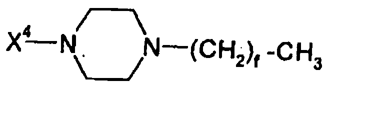 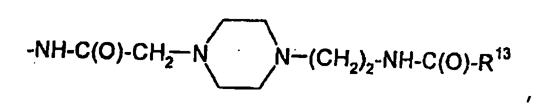 ou -C(O)-NHR¹², où X⁴ est, indépendamment pour chaque occurrence, -C(O)-, -NH-C(O)- ou -CH₂-, et où f est, indépendamment pour chaque occurrence, un entier de 1 à 29 inclus ; chacun de R² et R³ est indépendamment choisi dans le groupe constitué par H, alkyle en C₁-C₃₀, alcényle en C₂-C₃₀, phényl-alkyle en C₁-C₃₀, naphtyl-alkyle en C₁-C_{30,} hydroxy-alkyle en C₁-C_{30,} hydroxy-alcényle en C₂-C₃₀, hydroxyphényl-alkyle en C₁-C₃₀ et hydroxynaphtyl-alkyle en C₁-C₃₀ ; ou l'un de R² et R³ est 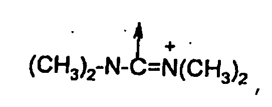 acyle en C₁-C₃₀, alkylsulfonyle en C₁-C₃₀, C(O)X⁵, 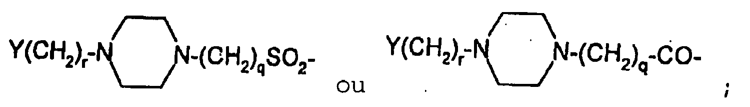 où
Y est H, OH ou NH₂ ; r est 0 à 4 ; q est 0 à 4 ; et X⁵ est alkyle en C₁-C₃₀, alcényle en C₂-C₃₀, phényl-alkyle en C₁-C₃₀, naphtyl-alkyle en C₁-C₃₀, hydroxy-alkyle en C₁-C₃₀, hydroxy-alcényle en C₂-C₃₀, hydroxyphényl-alkyle en C₁-C₃₀ ou hydroxynaphtyl-alkyle en C₁-C₃₀ ;
et
chacun de R¹² et R¹³ est, indépendamment pour chaque occurrence, alkyle en C₁-C₃₀ :
à la condition qui :
(i) ledit composé ne soit pas
(Ser⁸, β-Ala³⁵)hGLP-1(7-36)NH₂ (SEQ ID NO:779) ;
ou un sel pharmaceutiquement acceptable de ce composé.

2. Composé selon la revendication 1, ledit composé étant :
(Ser⁸, Aib³⁵)hGLP-1(7-36)NH₂ ; (SEQ ID NO:767)
(Abu⁸, β-Ala³⁵)hGLP-1(7-36)NH2 ; (SEQ ID NO:768)
(Val⁸, β-Ala³⁵)hGLP-1(7-36)NH2 ; (SEQ ID NO:769)
(β-Ala^{8,35})hGLP-1(7-36)NH₂ ; (SEQ ID NO:770)
(Abu⁸, Aib³⁵)hGLP-1(7-36)NH2 ; (SEQ ID NO:771)
(Val⁸, Aib³⁵)hGLP-1(7-36)NH2 ; (SEQ ID NO:772)
ou (β-Ala⁸,Aib³⁵)hGLP-1(7-36)NH₂ ; (SEQ ID NO:773)
ou un sel pharmaceutiquement acceptable de celui-ci.

3. Composé selon la revendication 2, ledit composé étant :
(Ser⁸, Aib³⁵)hGLP-1(7-36)NH₂ ; (SEQ ID NO:767)
(Abu⁸, β-Ala³⁵)hGLP-1(7-36)NH₂ ; (SEQ ID NO:768)
ou (Abu⁸, Aib³⁵)hGLP-1(7-36)NH₂ ; (SEQ ID NO:771)
ou un sel pharmaceutiquement acceptable de celui-ci.

4. Composé selon la revendication 3, ledit composé étant :
(Ser⁸, Aib³⁵)hGLP-1(7-36)NH2 ; (SEQ ID NO:767)
ou un sel pharmaceutiquement acceptable de celui-ci.

5. Composition pharmaceutique comprenant une quantité effective d'un composé selon la revendication 1 ou un sel pharmaceutiquement acceptable de celui-ci et un support ou un diluant pharmaceutiquement acceptable.

6. Composition pharmaceutique selon la revendication 5, dans laquelle ledit composé est :
(Ser⁸, β-Aib³⁵)hGLP-1(7-36)NH₂ ; (SEQ ID NO:767)
(Abu⁸, β-Ala³⁵)hGLP-1(7-36)NH₂ ; (SEQ ID NO:768)
(Val⁸, β-Ala³⁵)hGLP-1(7-36)NH₂ ; (SEQ ID NO:769)
(β-Ala^{8,35})hGLP-1(7-36)NH₂ ; (SEQ ID NO:770)
(Abu⁸, Aib³⁵)hGLP-1(7-36)NH₂ ; (SEQ ID NO:771)
(Val⁸, Aib³⁵)hGLP-1(7-36)NH₂ ; (SEQ ID NO:772)
ou (β-Ala³⁵,Aib³⁵)hGLP-1(7-36)NH₂ (SEQ ID NO:773)
ou un sel pharmaceutiquement acceptable de celui-ci.

7. Composition pharmaceutique selon la revendication 6, dans laquelle ledit composé est :
(Seᵣ⁸, β-Aib³⁵)hGLP-1(7-36)NH₂ ; (SEQ ID NO:767)
(Abu⁸, β-Ala³⁵)hGLP-1(7-36)NH₂; (SEQ ID NO:768)
ou (Abu⁸, Aib³⁵)hGLP-1(7-36)NH₂ ; (SEQ ID NO:771)
ou un sel pharmaceutiquement acceptable de celui-ci.

8. Composition pharmaceutique selon la revendication 7, dans laquelle ledit composé est :
(Ser⁸, Aib³⁵)hGLP-1(7-36)NH₂; (SEQ ID NO:767)
ou un sel pharmaceutiquement acceptable de celui-ci.

9. Utilisation d'une quantité effective d'un composé selon là revendication 1 ou d'un sel pharmaceutiquement acceptable de celui-ci dans la préparation d'un médicament pour déclencher un effet agoniste à partir d'un récepteur GLP-1 dans un sujet en ayant besoin.

10. Utilisation d'une quantité effective d'un composé selon la revendication 1 ou d'un sel pharmaceutiquement acceptable de celui-ci dans la préparation d'un médicament pour traiter une maladie choisie dans le groupe constitué par le diabète de type I, le diabète de type II, l'obésité, les glucagonomes, les troubles sécrétoires des voies aériennes, un trouble métabolique, l'arthrite, l'ostéoporose, une maladie du système nerveux central, la resténose et la maladie neurodégénérative, dans un sujet en ayant besoin.

11. Utilisation selon la revendication 10, dans laquelle ladite maladie est le diabète de type I ou le diabète de type II.

12. Utilisation selon l'une quelconque des revendications 9 à 11, dans laquelle ledit composé est :
(Ser⁸, Aib³⁵)hGLP-1(7-36)NH₂ ; (SEQ ID NO:767)
(Abu⁸, β-Ala³⁵)hGLP-1(7-36)NH₂ ; (SEQ ID NO:768)
(Val⁸, β-Ala³⁵)hGLP-1(7-36)NH₂ ; (SEQ ID NO:769)
(β-Ala^{8,35})hGLP-1(7-36)NH₂ ; (SEQ ID NO:770)
(Abu⁸, Aib³⁵)hGLP-1(7-36)NH₂ ; (SEQ ID NO:771)
(Val⁸, Aib³⁵)hGLP-1(7-36)NH₂ ; (SEQ ID NO:772)
ou (β-Ala⁸,Aib³⁵)hGLP-1(7-36)NH₂ ; (SEQ ID NO:773)
ou un sel pharmaceutiquement acceptable de celui-ci.

13. Utilisation selon la revendication 12, dans laquelle ledit composé est :
(Ser⁸, Aib³⁵)hGLP-1(7-36)NH₂ ; (SEQ ID NO:767)
(Abu⁸, β-Ala³⁵)hGLP-1(7-36)NH₂ ; (SEQ ID NO:768)
ou (Abu⁸, Aib³⁵)hGLP-1(7-36)NH₂ ; (SEQ ID NO:771)
ou un sel pharmaceutiquement acceptable de celui-ci.

14. Utilisation selon la revendication 13, dans laquelle ledit composé est :
(Seᵣ⁸, Aib³⁵)hGLP-1(7-36)NH₂ ; (SEQ ID NO:767)
ou un sel pharmaceutiquement acceptable de celui-ci.


### SEQUENCE LISTING

<110> SOCIETE DE CONSEILS DE RECHERCHES ET D'APPLICATIONS
   SCIENTIFIQUES, S.A.S
   DONG, ZHENG ZIN
<120> ANALOGUES OF GLP-1
<130> 129P-PCT2
<160> 781
<140>
   <141>
<150> 60/449,203
   <151> 2003-02-19
<170> PatentIn version 3.2
<210> 767
   <211> 30
   <212> PRT
   <213> Artificial Sequence
<220>
   <223> Description of Artificial Sequence: Synthetic modified hGLP-1 peptide
<220>
   <221> MOD_RES
   <222> (29).. (29)
   <223> Aib
<220>
   <223> c-term amidation
<400> 767
   His Ser Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Xaa Arg
               20                  25                  30
<210> 768
   <211> 30
   <212> PRT
   <213> Artificial Sequence
<220>
   <223> Description of Artificial Sequence: Synthetic modified hGLP-1 peptide
<220>
   <221> MOD_RES
   <222> (2)..(2)
   <223> Abu
<220>
   <221> MOD_RES
   <222> (29)..(29)
   <223> Beta-Ala
<220>
   <223> c-term amidation
<400> 768
   His Xaa Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Xaa Arg
               20                  25                  30
<210> 769
   <211> 30
   <212> PRT
   <213> Artificial Sequence
<220>
   <223> Description of Artificial Sequence: Synthetic modified hGLP-1 peptide
<220>
   <221> MOD_RES
   <222> (29)..(29)
   <223> Beta-Ala
<220>
   <223> c-term amidation
<400> 769
   His Val Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Xaa Arg
               20                  25                  30
<210> 770
   <211> 30
   <212> PRT
   <213> Artificial Sequence
<220>
   <223> Description of Artificial Sequence: Synthetic modified hGLP-1 peptide
<220>
   <221> MOD_RES
   <222> (2)..(2)
   <223> Beta-Ala
<220>
   <221> MOD_RES
   <222> (29).. (29)
   <223> Beta-Ala
<220>
   <223> c-term amidation
<400> 770
   His Xaa Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Xaa Arg
               20                  25                  30
<210> 771
   <211> 30
   <212> PRT
   <213> Artificial Sequence
<220>
   <223> Description of Artificial Sequence: Synthetic modified hGLP-1 peptide
<220>
   <221> MOD_RES
   <222> (2)..(2)
   <223> Abu
<220>
   <221> MOD_RES
   <222> (29)..(29)
   <223> Aib
<220>
   <223> c-term amidation
<400> 771
   HIs Xaa Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Xaa Arg
               20                  25                  30
<210> 772
   <211> 30
   <212> PRT
   <213> Artificial Sequence
<220>
   <223> Description of Artificial Sequence: Synthetic modified hGLP-1 peptide
<220>
   <221> MOD_RES
   <222> (29)..(29)
   <223> Aib
<220>
   <223> c-term amidation
<400> 772
   His Val Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Xaa Arg
               20                  25                  30
<210> 773
   <211> 30
   <212> PRT
   <213> Artificial Sequence
<220>
   <223> Description of Artificial Sequence: Synthetic modified help-1 peptide
<220>
   <221> MOD_RES
   <222> (2)..(2)
   <223> Beta-Ala
<220>
   <221> MOD_RES
   <222> (29).. (29)
   <223> Aib
<220>
   <223> c-term amidation
<400> 773
   His Xaa Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Xaa Arg
               20                  25                  30